Europäisches Patentamt

European Patent Office

Office européen des brevets

(11) Publication number: **0 257 510**
**A2**

(12) **EUROPEAN PATENT APPLICATION**

(21) Application number: **87111902.0**

(22) Date of filing: **17.08.87**

(51) Int. Cl.⁴: **C11C 3/00** , C07C 51/00 , C07C 69/00

(30) Priority: **21.08.86 JP 195883/86**
            **26.12.86 JP 315197/86**

(43) Date of publication of application:
      **02.03.88 Bulletin 88/09**

(84) Designated Contracting States:
      **DE NL**

(71) Applicant: **LION CORPORATION**
      **3-7, Honjo 1-chome**
      **Sumida-ku Tokyo(JP)**

(72) Inventor: **Hatakeyama, Norio**
      **1229-242, Oji-cho**
      **Chiba-shi Chiba(JP)**
      Inventor: **Tano, Tetsuo**
      **6-11-2-305, Ryoke**
      **Urawa-shi Saitama(JP)**
      Inventor: **Kitano, Kyozo**
      **2-1-3-402, Akitsu**
      **Narashino-shi Chiba(JP)**

(74) Representative: **Henkel, Feiler, Hänzel &**
      **Partner**
      **Möhlstrasse 37**
      **D-8000 München 80(DE)**

(54) **Method for modifying unsaturated fatty acid or ester thereof.**

(57) A method for modifying an unsaturated fatty acid or an ester thereof characterized by comprising the steps of:

adding 1 to 50 parts by weight of an acid and 0.1 to 10 parts by weight of hydrogen peroxide to 100 parts by weight of an unsaturated fatty acid having 8 to 30 carbon atoms or an ester thereof with a monohydric or polyhydric alcohol, and

allowing the resultant mixture to react at a temperature of 20°C to 100°C to decrease the degree of unsaturation of the unsaturated fatty acid or the ester thereof.

EP 0 257 510 A2

## METHOD FOR MODIFYING UNSATURATED FATTY ACID OR ESTER THEREOF

The present invention relates to a method for modifying an unsaturated fatty acid or an ester thereof. More specifically, it relates to a method for decreasing the degree of unsaturation of an unsaturated fatty acid having 8 to 30 carbon atoms or an ester thereof with a monohydric or polyhydric alcohol.

Heretofore, hydrogenation has been generally carried out to obtain a saturated fatty acid ester from an unsaturated fatty acid ester. More specifically, the hydrogenation is carried out in the presence of, for example, a Pt or Cu catalyst at a reaction temperature of 180°C to 200°C under a hydrogen pressure of 2 to 4 kg/cm$^2$. However, since the hydrogenation is carried out under a high temperature and a high pressure, the hydrogenation apparatus must be disadvantageously large and complicated and the conventional hydrogenation is disadvantageous from the viewpoints of safety and energy consumption. Furthermore, since hydrogenation is a gas-liquid reaction, special care must be taken with the dispersibility and contact method of the hydrogen, to effect a stable reaction, and thus the reaction operation becomes disadvantageously complicated.

Accordingly, the objects of the present invention are to eliminate the above-mentioned disadvantages in the conventional hydrogenation of unsaturated fatty acids or esters thereof and to provide a method for decreasing the degree of unsaturation of an unsaturated fatty acid or an ester thereof simply and under a stable condition at a temperature of 100°C or less under a normal pressure by using a simple apparatus.

Other objects and advantages of the present invention will be apparent from the following description.

In accordance with the present invention, there is provided a method for modifying an unsaturated fatty acid or an ester thereof characterized by comprising the steps of:

adding 1 to 50 parts by weight of an acid and 0.1 to 10 parts by weight of hydrogen peroxide to 100 parts by weight of an unsaturated fatty acid having 8 to 30 carbon atoms or an ester thereof with a monohydric or polyhydric alcohol, and allowing the resultant mixture to react at a temperature of 20°C to 100°C to decrease the degree of unsaturation of the unsaturated fatty acid or the ester thereof.

The unsaturated fatty acids usable in the present invention are those having 8 to 30 carbon atoms, preferably 10 to 20 carbon atoms, and having at least one unsaturated bond, preferably 1 to 3 unsaturated bonds, in the molecule.

The esters of the unsaturated fatty acids with alcohols usable in the present invention are esters of the unsaturated fatty acids with monohydric alcohols, preferably with monohydric alcohols with 1 to 8 carbon atoms; mono-or di-esters thereof with dihydric alcohols such as ethylene glycol, propylene glycol; mono-, di-, and tri-esters thereof with glycerol; and partial and full esters thereof with polyhydric alcohols such as pentaerithritol and sorbitol.

According to the present invention, the above-mentioned unsaturated fatty acids and the esters thereof may be used above or in any mixtures thereof. Furthermore, these unsaturated fatty acids and the esters thereof may be used in the form of mixtures thereof with saturated fatty acids or the esters thereof. Examples of such saturated fatty acids and the esters thereof are natural fatty acids such as palm fatty acids, tallow fatty acids, and coconut fatty acids and the esters thereof with monohydric and polyhydric alcohols.

According to the present invention, 1 to 50 parts by weight, preferably 10 to 30 parts by weight, of the acid, and 1 to 10 parts by weight, preferably 2 to 7 parts by weight, of hydrogen peroxide in terms of 100% H$_2$O$_2$ , both based upon 100 parts by weight of the above-mentioned unsaturated fatty acids and the esters thereof, are used as an aqueous solution. More preferably, this solution further includes 50 parts by weight or less of lower alcohols, preferably 10 to 30 parts by weight, based on 100 parts by weight of the above-mentioned unsaturated fatty acids and the esters thereof.

The acids usable in the present invention may include, for example, mineral acids such as sulfuric acid, phosphoric acid, and hydrochloric acid; benzene sulfonic acid; and alkylbenzene sulfonic acids with a C$_1$ - C$_{14}$ alkyl group. Of these acids, the use of sulfuric acid or an alkylbenzene sulfonic acid is preferable in the practice of the present invention.

The hydrogen peroxide usable in the present invention is preferably used in the form of an aqueous solution having 31.2 to 50 percent by weight content of hydrogen peroxide.

The lower alcohols optionally used in the present invention may include those having 1 to 6 carbon atoms such as methanol, ethanol, n-propanol, and isopropanol, preferably methanol and ethanol.

According to the present invention, the modification of the unsaturated fatty acids or the esters thereof can be carried out by adding the above-mentioned components to the unsaturated fatty acids or the esters thereof under a non-aqueous system or in the presence of 10 parts by weight or less, based upon 100 parts by weight of the resultant mixture, of water. Thus, unsaturated bonds in the unsaturated fatty acids or the esters thereof are converted to saturated bonds. The modification reaction may be carried out, while mixing, at a temperature of 20°C to 100°C, preferably 45°C to 80°C, for 10 minutes to 4 hours, preferably 30 minutes to 2 hours. When the reaction is carried out at a temperature of less than 20°C, the desired modification may not be effected and thus the desired saturated fatty acids or the esters thereof not obtained. Conversely, when the reaction is carried out at a temperature of more than 100°C, saturated fatty acids or esters having dark color and bitter odor are obtained. Furthermore, an extreme vaporization of the alcohol occurs necessitating the use of a pressurized apparatus. This is not preferable from the industrial point of view, since high pressure operations are always dangerous.

As mentioned above, according to the present invention, the unsaturated fatty acids and the esters thereof can be advantageously modified into saturated fatty acids and the esters thereof by a very simple treatment. Accordingly, the problems of the conventional techniques, e.g., the use of a large sealed apparatus, and need for complicated processes, safety problems, and an unpreferable energy consumption, can be solved, and therefore, the present method is suitable for use as an industrial process for the production of saturated fatty acids or the esters thereof.

Furthermore, according to the present invention, the ratio of saturated/unsaturated fatty acids or the esters thereof can be easily and freely controlled to a desired value by changing the addition ratio of the acid/hydrogen peroxide. Accordingly, oil and fats having the desired properties can be readily produced on a commercial scale and, therefore, the various fundamental characteristics thereof as a surfactant starting material, such as surface tension, penetrating power, foaming power, and rinsing ability, can be easily adjusted. Therefore, the modification technique of the present invention can be widely used in various fields including detergent industries.

Namely, according to the present invention, although the detailed mechanism of the reaction is not fully understood, there is no scission of the molecule at the unsaturated bonds, and polymerization does not occur, because of the low reaction temperature and, therefore, the unsaturated bonds are blocked with, for example, hydrogen atoms and methoxy groups. Furthermore, when the modification reaction of the present invention is carried out in the presence of a lower alcohol, the optimum desired starting materials of surfactants can be obtained without causing a scission of the ester bonds in the fatty acid esters and with an easy reaction temperature control.

## EXAMPLE

The present invention now will be further illustrated by, but is by no means limited to, the following example, in which all parts are expressed on a weight basis unless otherwise specified.

### Example 1

A 59 g amount of methyl oleate having an iodine value (i.e., "I.V.") of 84 and 941 g of methyl stearate having an I.V. of less than 0.1 were charged into a 2 liter mixing vessel, followed by adding 35 g of a 35% aqueous hydrogen peroxide and 50 g concentrated sulfuric acid. The mixture was then mixed at a temperature of 80°C for about one hour, while stirring. The resultant mixture was then allowed to stand for separation at the same temperature, and the separated oil layer was washed with warm water. The water was topped from the oil layer, followed by drying over sodium sulfate.

Thus, the desired straight-chain saturated fatty acid and the ester thereof were obtained. The iodine value of the main product was decreased by 0.3 when measured by an Wij's method.

### Example 2

The reaction of Example 1 was repeated in the presence of an acid and hydrogen peroxide by changing a mixing ratio of methyl oleate and methyl stearate.

The reaction conditions and the results are shown in Table 1. In Table 1, the addition amounts of the acid and hydrogen peroxide are parts by weight based on 100 parts by weight of the starting esters. As is clear from the results shown in Table 1, the degree of unsaturation of the unsaturated fatty acid esters can be remarkably decreased.

Table 1

| Run No. | Comparative | | | | | Present Invention | | | | |
|---|---|---|---|---|---|---|---|---|---|---|
| | 1 | 2 | 3 | 4 | 5 | 6 | 7 | 8 | 9 | 10 |
| I.V. of Starting Material | ← 5 → | | | 1 | 0.6 | 4 | 6 | 10 | 30 | 52 |
| Acid Kind | ← Conc. Sulfuric acid → | | | Conc. Sulfuric acid | | Phosphoric acid | LAS acid* | Conc. Sulfuric acid | | |
| Amount added | 1 | 0.5 | 5 | 1.5 | 1 | 10 | 3 | 10 | 16 | 30 |
| Amount of $H_2O_2$ added | 0.05 | 5 | 1 | 0.2 | 0.1 | 3 | 1 | 2 | 3 | 7 |
| Mixing Temp. (°C) | 50 | 70 | 10 | 40 | 50 | 70 | 65 | 30 | 55 | 40 |
| I.V. of Ester after Treatment | 4.8 | 4.7 | 4.9 | 0.1 | 0 | 0.2 | 0.3 | 0.5 | 3 | 5 |

*: Linear alkylbenzene sulfonic acid

## Example 3

A 1 kg amount of palm fatty acid methyl ester having an average molecular weight of 282, an I.V. of 52, and a ratio of saturated/unsaturated esters of 50/50 was charged into a 2 liter mixing vessel, followed by adding 50 g of concentrated sulfuric acid, 57 g of a 35% aqueous hydrogen peroxide, and 150 g of methanol. The mixture was allowed to react at a temperature of 75°C for about 1 hour while stirring.

The resultant mixture was washed twice by adding 100 parts of warm water having a temperature of 60°C to 70°C, based on 100 parts of the mixture. The water and methanol were topped from the mixture, followed by drying over sodium sulfate.

Thus, the desired modified methyl ester of palm fatty acid was obtained. The I.V. of the resultant modified methyl ester was 35 as measured by an Wij's method.

## Example 4

Various fatty acid esters were treated in the same manner as in Example 3, except that the starting esters and the treatment conditions were changed as shown in Table 2.

The results are shown in Table 2. From the results shown in Table 2, it can be seen that the degree of unsaturation of the unsaturated fatty acid can be remarkably decreased.

Table 2

| | Comparative | | | | Present Invention | | | | |
|---|---|---|---|---|---|---|---|---|---|
| Run No. | 1 | 2 | 3 | 4 | 5 | 6 | 7 | 8 | 9 |
| Starting Ester — Kind | Methyl Ester of Palm Fatty Acid → | | | | | | | | Mixture of Methyl Oleate and Methyl Stearate |
| I.V. | 52 | 52 | 52 | 52 | 52 | 5 | 10 | 30 | 1 |
| Acid — Kind | Concentrated Sulfuric Acid → | | | | | | LAS acid + Conc. Sulfuric Acid → | | |
| Acid — Amount Added | 10 | 0.5 | 15 | 20 | 45 | 10 | 20 | 10 | 3 |
| H₂O₂ Addition Amount | 2 | 5 | 0.5 | 7 | 9 | 2 | 4 | 8 | 1 |
| Lower Alcohol — Kind | Methanol → | | | | | Ethanol + Methanol → | | | |
| Lower Alcohol — Amount Added | 15 | 10 | 15 | 3 | 30 | 10 | 20 | 10 | 7 |
| Mixing Temperature (°C) | 30 | 70 | 75 | 10 | 70 | 55 | 50 | 80 | 70 |
| I.V. of Ester after Treatment | 49 | 49 | 51 | 48 | 2 | 0.5 | 1 | 4 | 0 |

## Claims

1. A method for modifying an unsaturated fatty acid or an ester thereof characterized by comprising the steps of:

adding 1 to 50 parts by weight of an acid and 0.1 to 10 parts by weight of hydrogen peroxide to 100 parts by weight of an unsaturated fatty acid having 8 to 30 carbon atoms or an ester thereof with a monohydric or polyhydric alcohol, and

allowing the resultant mixture to react at a temperature of 20°C to 100°C to decrease the degree of unsaturation of the unsaturated fatty acid or the ester thereof.

2. A method as claimed in claim 1, wherein the reaction of the mixture is carried out in the presence of 50 parts by weight or less of lower alcohols, based upon 100 parts of the unsaturated fatty acid or the ester thereof, of a lower alcohol at a temperature of 40°C to 80°C.

3. A method as claimed in claim 1 or 2, wherein said acid is a mineral acid.

4. A method as claimed in claim 1 or 2, wherein said acid is benzene sulfonic acid or an alkylbenzene sulfonic acid with a $C_1$ to $C_{14}$ alkyl group.

5. A method as claimed in claim 2, wherein said lower alcohol is an alcohol having 1 to 6 carbon atoms.